# EUROPEAN PATENT APPLICATION

(11) **EP 1 350 460 A2**
(43) Date of publication of application: **08.10.2003**
(21) Application number: 03006266.5
(22) Date of filing: 21.03.2003
(51) Int. Cl.: A61B 5/00

(54) **Vital sign detection sensor and sensor controlling device**

(30) Priority: 25.03.2002 JP 2002083073
(71) Applicant: MATSUSHITA ELECTRIC INDUSTRIAL CO., LTD., Kadoma-shi, Osaka 571-8501 (JP)
(72) Inventor: Mori, Yasuhiro, Izumi-shi, Osaka, 594-0073 (JP)
(74) Representative: Balsters, Robert

(57) **Abstract**

A sensor device 10A is comprised of a power generating unit 111A that takes in energy externally (a power carrier radio wave) and generates direct current power from such energy, a vital sign detecting unit 112A that detects a vital sign of a user while being supplied with the direct current power from the power generating unit 111A, and a vital sign transmitting unit 116A that transmits the detected vital sign cordlessly while being supplied with the direct current power from the power generating unit 111A.

## Description

### BACKGROUND OF THE INVENTION

### (1) Field of the Invention

The present invention relates to a portable vital sign detection sensor that is used attached to a human body, and to a sensor controlling device that receives a vital sign transmitted from such vital sign detection sensor and controls operations of the vital sign detection sensor detecting a vital sign.

### (2) Description of the Related Art

In response to a demand for using a vital sign detection sensor for such purposes as health check, recent years has witnessed the development of vital sign detection sensors for detecting vital signs which are used attached to a detection position of a user. Moreover, against the backdrop where the size of electronic devices is increasingly reduced, there is an ongoing trend toward the downsizing of vital sign detection sensors.

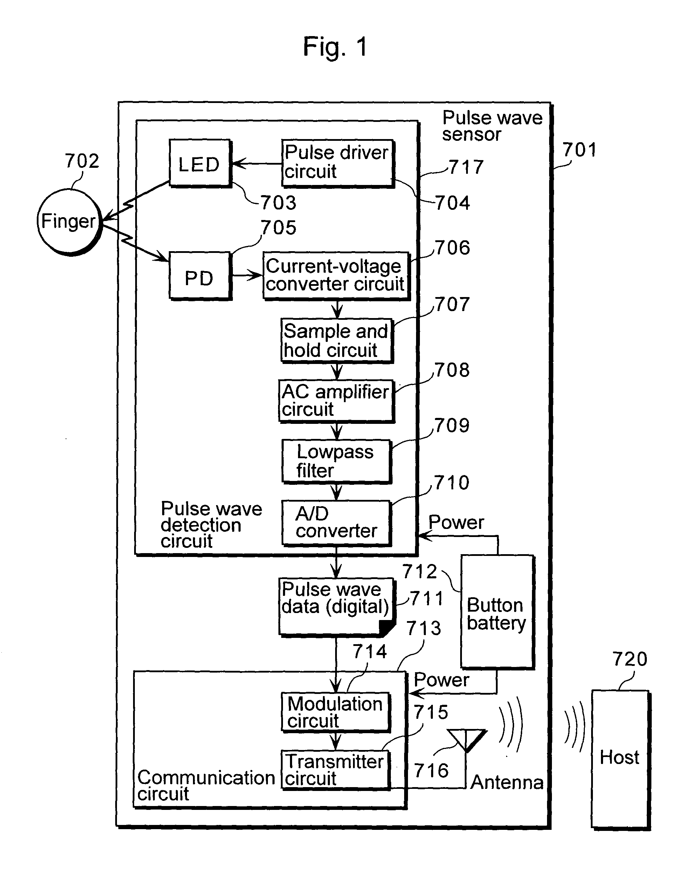

A pulse wave sensor illustrated in Fig.1 is an example of such a downsized vital sign detection sensor. This pulse wave sensor is disclosed in the technical research report MBE2000-73 of the Institute of Electronics, Information and Communication Engineers.

Fig.1 is a diagram showing a circuit configuration of a system which includes the prior pulse wave sensor disclosed in the above report.

A pulse wave sensor 701 illustrated in Fig.1, which is used attached to a human body (a finger), comprises a pulse wave detection circuit 717, a communication circuit 713, an antenna 716, a button battery 721 that supplies power to each unit of the pulse wave detection circuit 717 and the communication circuit 713. The pulse wave detection circuit 717 includes a light emitting diode (LED) 703, a pulse driver circuit 704, a photo diode (PD) 705, a current-voltage converter circuit 706, a sample and hold circuit 707, an alternating current (AC) amplifier circuit 708, a lowpass filter 709, and an A/D converter 710. The communication circuit 713 is made up of a modulation circuit 714 and a transmitter circuit 715. The pulse wave sensor 701 is configured to wirelessly transmit a vital sign to a host by radio waves.

The LED 703 of the pulse wave sensor 701 with the above configuration is pulse-driven by the pulse driver circuit 704 and emits a flashing light. A part of the light emitted from the LED 703 is reflected on the skin surface of a finger 702 and a part of the light which goes into the internal skin is reflected at a capillary artery. The PD 705 receives both of such reflected lights and outputs a photoelectric current. The current-voltage converter circuit 706 converts such photoelectric current into a voltage. Since this voltage is a pulse wave, the sample and hold circuit 707 converts the pulse wave into a continuous wave. Then, the AC amplifier circuit 708 cuts off the reflection on the skin surface (mostly a DC component) so as to extract only a pulse wave (an AC component). Next, the lowpass filter 709 shapes the pulse wave, and the A/D converter 710 converts the pulse wave, which is analog data, into digital data. The modulation circuit 714 in the communication circuit 713 modulates a radio wave by digitalized pulse wave data 711, and the transmitter circuit 715 converts a high-frequency electric signal upon which the pulse wave data is superimposed into a radio wave, which is then transmitted via the antenna 716.

A host 720 receives the radio wave, demodulates the pulse wave data superimposed upon such radio wave and shows the pulse wave on a display not illustrated in the figure. The host 720 includes a CPU and a storage device inside. It is possible for such CPU to perform a variety of processing which includes the determination of heart rate on the basis of the received pulse wave data so as to show the determined heart rate onto a display. For instance, by accumulating the heart rate for a long period of time and performing statistical processing for such heart rate, it is possible to measure a cyclical variation of the heart rate over a long span of time. If a clearer heart wave can be obtained, it becomes possible to utilize the host 720 as an unsophisticated unit for finding a heart disease and to give a notice to a patient with a heart disease at an earlier stage. In other words, it is possible to perform a wide range of processing on the basis of pulse wave data, using a necessary application program.

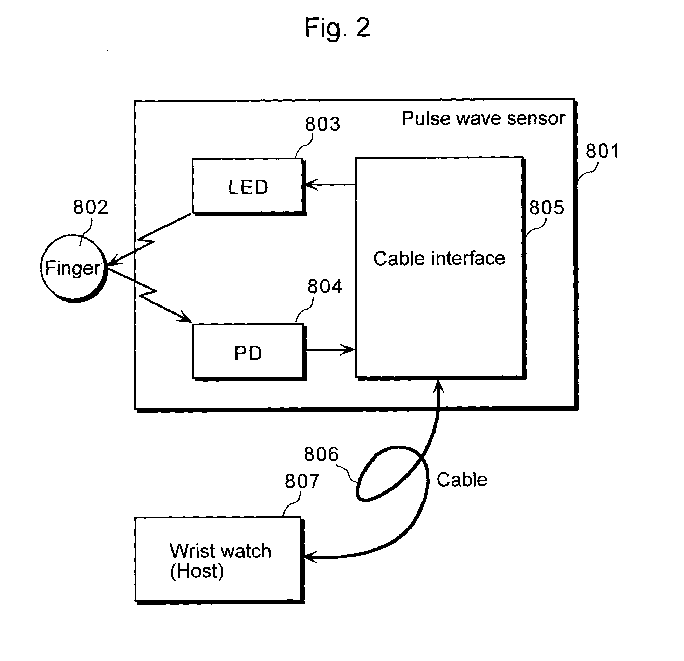

Fig.2 illustrates another prior pulse wave sensor, which is connected to a host. Such host-connected pulse wave sensor is comprised roughly with a pulse wave sensor 801 that measures a pulse wave, a wrist watch (host) 807 having a data processing unit that processes the measured data and a cable 806 that connects the pulse wave sensor 801 and the wrist watch 807. The cable 806 is responsible for supplying power from the wrist watch 807 to the pulse wave sensor 801, transmitting a control signal used by the wrist watch 807 to control the pulse wave sensor 801, transmitting data from the pulse wave sensor 801 to the wrist watch 807 and others.

Main constituent elements of the pulse wave sensor 801 are a light emitting diode (LED) 803, a photo diode (PD) 804 and a cable interface 805, all of which are driven by direct current power supplied from the wrist watch 807 via the cable 806. Data received by the photo diode (PD) 804 is transmitted to the wrist watch 807 via the cable interface 805 and the cable 806, and then processed by the wrist watch 807.

As is obvious, the pulse wave sensor 801, which includes the cable interface 805, the light emitting diode (LED) 803 and the photo diode (PD) 804 as its main constituent elements, has a smaller number of elements as well as a simpler configuration than the wireless pulse wave sensor presented as the first prior art. What should be noted is that such pulse wave sensor 801 does not include any battery, which is a significant advantage contributing greatly to the downsizing of pulse wave sensors.

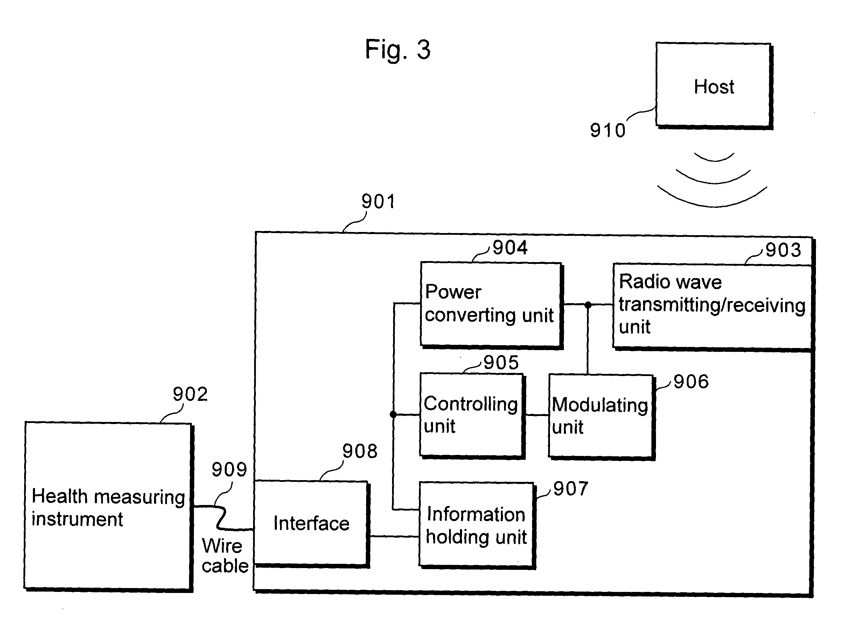

As another prior art (the third prior art), Fig.3 shows a circuit configuration of a system including a health measuring instrument connected to a communication circuit. Such prior system is disclosed in the Japanese Laid-Open Patent No. 2000-196510.

This system is comprised of a health measuring instrument 902 that measures a person's physical condition, a communication circuit 901 that is connected to the health measuring instrument 902 by a wire cable 909 and a host 910 that communicates with the communication circuit 901 by radio so as to exchange data.

The communication circuit 901, which is inserted into the health measuring equipment 902 connected by the wire cable 909, is a circuit for relaying and transferring health data measured by the health measuring equipment 902 to the host 910. Such communication circuit 901 includes a radio wave transmitting/receiving unit 903, a power converting unit 904, a controlling unit 905, a modulating unit 906, an information holding unit 907 and an interface 908.

The radio wave transmitting/receiving unit 903 receives a power carrier radio wave transmitted from the host 910 in compliance with the electromagnetic induction method, and generates induced electric power. Then, the power converting unit 904 converts the induced electric power generated by the radio wave transmitting/receiving unit 903 into direct current power. The modulating unit 906 and the controlling unit 905 are driven by direct current power supplied from the power converting unit 904. Meanwhile, the information holding unit 907 and the interface 908 are configured to be driven by direct current power supplied from the health measuring instrument 902 via the wire cable 909.

When obtaining data, the health measuring instrument 902 supplies power to the interface 908 and the information holding unit 907 of the communication circuit 901 so as to drive them, and writes the obtained data to the information holding unit 907 via the interface 908. This indicates that, whenever the health measuring instrument 902 obtains data and such data needs to be written to the communication circuit 901, it is possible for the data to be written regardless of whether the controlling unit 905 and the modulating unit 906 are being driven or not.

When transmitting the data to the host 910, the health measuring instrument 902 inserted with the communication circuit 901 is brought into an area within which the power carrier radio wave transmitted by the host 910 is receivable. Subsequently, the radio wave transmitting/receiving unit 903 of the communication circuit 901 receives the radio wave and outputs an electromotive force included in such received radio wave to the power converting unit 904, which then converts the electromotive force into direct current power. The controlling unit 905 and the modulating unit 906 are driven by power generated by the power converting unit 904 so that the controlling unit 905 reads the data from the information holding unit 907 driven by direct current power supplied from the health measuring instrument 902, and the modulating unit 906 modulates a high-frequency signal by the data. Then, the radio wave transmitting/receiving unit 903 transmits the data to the host 910 by emitting the radio wave superimposed with the data. As a result, since a wireless data communication is realized between the health measuring instrument 902 inserted with the communication circuit 901 and the host 910, and electric power is generated on the part of the communication circuit 901 in accordance with the electromagnetic induction method, the communication circuit 901 does not require any power source including a battery. This consequently leads to a significant reduction in the size of the communication circuit 901.

However, the configurations of the wireless pulse wave sensor (the first prior art) and the cable-type pulse wave sensor (the second prior art) have problems described below.

A pulse wave sensor is attached to a human finger and therefore needs to be so small as not to be obtrusive when the user waves his/her hand, for example.

However, regarding the wireless pulse wave sensor, i.e. the first prior art, since it employs a battery as a power source, the size of the battery hinders the sensor device from being reduced in size to a desirable level.

A possible solution to this problem is the use of a smaller battery instead of a button battery. However, since a small battery has a reduced battery capacity, the operating hours of the sensor device is extremely reduced, which necessitates frequent battery changes. As a result, the number of times a battery needs to be changed is increased, producing another problem that a battery cost and a sensor operating cost are increased.

Concerning the third prior art, although the communication circuit itself does not include any battery which would cause the above problem, the health measuring instrument inserted with such communication circuit has a power source for driving the interface and the information holding unit of the communication circuit. Therefore, when seeing the integration of such communication circuit and such health measuring instrument as a single sensor device, it is impossible to reduce the size of such sensor device as in the case of the first prior art.

Meanwhile, the configuration of the cable-type pulse wave sensor presented as the second prior art does not include any battery, and therefore there is a fewer obstacles for downsizing. However, the problem with the configuration of this pulse wave sensor is the cable that connects the wrist watch with the sensor.

To be more specific, this pulse wave sensor is configured to be attached to a finger, because a finger has a thinner skin and therefore serves as a suitable position from which a pulse wave is most easily detected. However, since hand fingers are frequently moved, there is a possibility that a pulse wave fails to be obtained because of the sensor attached at a misaligned angle on the finger or detached, if the cable is pulled by something while the user is exercising, for example. Furthermore, it is troublesome for the user to always have to pay attention to the sensor device so that it will not be misaligned. Therefore, it is desirable that there is no cable, but the fact is that it is indispensable for supplying direct current power to each unit of the sensor device.

In other words, due to an embedded battery and the usage of a cable, the foregoing first to the third prior arts have the first problem that they cannot realize a downsized pulse wave sensor capable of reliably detecting a vital sign.

Another problem the first to the third prior arts have in common is that the sensor devices are not disposable or that it is difficult to make them disposable. More specifically, since a sensor device directly contacts a user's skin, it is more hygienic if the sensor device is replaced with a new one, just like a first-aid tape, when it gets dirty or after an appropriate time (about a few days later), considering sweat and other dirt. Accordingly, the user is required to purchase disposable sensor devices over a long period of time, indicating that their production cost should be inexpensive enough so that purchasing of such sensor devices will not be an enormous e conomic burden to the user. The reduction of the production cost which allows a lowered production cost of pulse wave sensors requires two-pronged approach which is: the reduction of manufacturing cost realized by a decreased number of constituent elements; and a decrease in the number of manufacturing steps at the assembly stage.

Concerning the wireless pulse wave sensor, a possible solution is to make it disposable according to its battery life. However, if a wireless pulse wave sensor needs to be replaced with a new one at a short cycle (about a few days), it is not easy to lower its production cost, given that it includes a battery. Regarding the cable-type pulse wave sensor, it may be possible to reduce its production cost, since it includes a smaller number of constituent elements. However, the production of cable-type pulse wave sensors involves complex steps for integrating a cable interface, an LED, a PD and others onto a board, indicating that it is difficult to reduce the number of such steps. Moreover, it is obvious from one's past experience that the contact area of a cable gets worn after frequent insertion and removal of such cable, which poses a durability issue on the part of a wrist watch (host).

To conclude, the foregoing first to the third prior arts have the second problem that they cannot provide a disposable vital sign detection sensor.

### SUMMARY OF THE INVENTION

In order to solve the above problems, the first object of the present invention is to provide a downsized vital sign detection sensor capable of reliably detecting a vital sign and a sensor controlling device that controls such sensor.

The second object of the present invention is to provide a disposable vital sign detection sensor and a sensor controlling device that controls such sensor.

In order to achieve the first object, a vital sign detection sensor that is portable and used attached to a human body, may comprise: a power generating unit operable to take in energy externally and generate direct current power from the energy; a vital sign detecting unit operable detect a vital sign of a user while being supplied with the direct current power from the power generating unit; and a vital sign transmitting unit operable to transmit the detected vital sign cordlessly while being supplied with the direct current power from the power generating unit. Accordingly, there is no need for a battery or a cable. As a result, it is possible for the present invention to provide a downsized vital sign detection sensor capable of reliably detecting a vital sign.

To be more specific, the vital sign detection sensor may be configured in a manner in which the energy is a power carrier radio wave, and the power generating unit is equipped with an antenna that receives the power carrier radio wave, and in which the energy is light energy, and the power generating unit includes a light-electricity converting unit operable to convert the light energy into electric energy.

Furthermore, the vital sign detection sensor may be configured in a manner in which the vital sign generating unit is equipped with an antenna that transmits the vital sign by radio, in which the vital sign generating unit includes an interface for transmitting the vital sign by using a body of the user as a transmission medium, and in which the vital sign generating unit includes an electricity-light converting unit operable to transmit the vital sign by lightwaves.

In order to achieve the second object, the vital sign detection sensor may be configured in a manner in which the vital sign detecting unit and at least a part of the power generating unit and the vital sign transmitting unit are integrated into one or more chips. Accordingly, since the manufacturing steps will be simplified and the manufacturing cost will be readily lowered, it is possible for the present invention to provide a disposable vital sign detection sensor.

More specifically, the vital sign detection sensor may further comprise a tape that has an adhesive side on which said one or more chips are attached and that is elastic, wherein said one or more chips are firmly fixed on a detection position of the user's body by winding the tape around the detection position, with a vital sign detecting side of said one or more chips contacting the detection position, and may be configured in a manner in which at least one of the power generating unit and the vital sign transmitting unit includes an antenna used for one of the following purposes: receiving the power carrier radio wave and transmitting the vital sign, and the antenna is placed in one of the following positions: around said one or more chips and on a non-adhesive side of the tape.

Furthermore, a sensor controlling device that receives a vital sign transmitted from the vital sign detection sensor with the above configuration and controls a vital sign detection operation of the vital sign detection sensor, may comprise: a power carrier radio wave transmitting unit operable to transmit a power carrier radio wave when the vital sign detection sensor detects the vital sign; a vital sign receiving unit operable to cordlessly receive the vital sign transmitted from the vital sign detection sensor; a memory unit operable to memorize the vital sign received by the vital sign receiving unit by an amount of a specified time period; and a controlling unit operable to control the power carrier radio wave transmitting unit transmitting the power carrier radio wave, from when the vital sign detection operation of the vital sign detection sensor starts to when the memory unit finishes memorizing the vital sign by the amount of the specified time period..

Similarly, the sensor controlling device may comprise: a control code transmitting unit operable to transmit by lightwaves a control code for making the vital sign detection sensor perform the vital sign detection operation; a vital sign receiving unit operable to cordlessly receive the vital sign transmitted from the vital sign detection sensor; a memory unit operable to memorize the vital sign received by the vital sign receiving unit by an amount of a specified time period; and a controlling unit operable to control the control code transmitting unit transmitting the control code, from when the vital sign detection operation of the vital sign detection sensor starts to when the memory unit finishes memorizing the vital sign by the amount of the specified time period..

Note that it is also possible to realize the present invention as a vital sign detection system comprising the vital sign detection sensor and the sensor controlling device, as a vital sign detection method that includes the characteristic units of the vital sign detection sensor as its steps, and as a program for having a personal computer or the like execute the steps performed by the sensor controlling device. It should be understood that it is also possible to widely distribute such program by storing it on recording media including a DVD.

Japanese patent application No. 2002-083073 filed March 25, 2002 is incorporated herein by reference.

### BRIEF DESCRIPTION OF THE DRAWINGS

These and other subjects, advantages and features of the invention will become apparent from the following description thereof taken in conjunction with the accompanying drawings that illustrate a specific embodiment of the invention. In the Drawings:
Fig.1 is a diagram showing a circuit configuration of a system including a conventional pulse wave sensor.
Fig.2 is a diagram showing a circuit configuration of a system including another conventional pulse wave sensor.
Fig.3 is a diagram showing a circuit configuration of a system including another conventional pulse wave sensor.
Figs.4A and 4B are diagrams showing an external view of an entire structure of a vital sign detection system 1A according to the first embodiment of the present invention.
Fig.5 is a diagram showing a cross-sectional structure of a sensor device 10A when seen from the sectional line A-A shown in Fig.4A.
Fig.6 is a diagram showing a circuit configuration of the sensor device 10A and a circuit configuration of a sensor controlling device 20A illustrated in Figs.4A and 4B.
Fig.7 is a flowchart illustrating an operation executed by a CPU 232 illustrated in Fig.6.
Figs.8A∼8G are waveform charts showing operations of major units illustrated in Fig.6 at vital sign detection times.
Fig.9 is a sequence diagram showing an exchange between the sensor device 10A and the sensor controlling device 20A.
Fig.10 is a diagram showing an external view of an entire structure of a vital sign detection system 1B according to the second embodiment of the present invention.
Fig.11 is a diagram showing a circuit configuration of a sensor device 10B and a circuit configuration of a sensor controlling device 20B illustrated in Fig.10.
Fig.12 is a sequence diagram showing an exchange between the sensor device 10B and the sensor controlling device 20B.
Fig.13 is a diagram showing an external view of an entire structure of a vital sign detection system 1C according to the third embodiment of the present invention.
Fig.14 is a diagram showing a cross-sectional structure of a sensor device 10C when seen from the sectional line B-B shown in Fig.13.
Fig.15 is a diagram showing a circuit configuration of the sensor device 10C and a circuit configuration of a sensor controlling device 20C illustrated in Fig.13.

### DESCRIPTION OF THE PREFERRED EMBODIMENTS

The flowing provides detailed explanations of the vital sign detection system according to the preferred embodiments of the present invention with reference to the figures.

### (The First Embodiment)

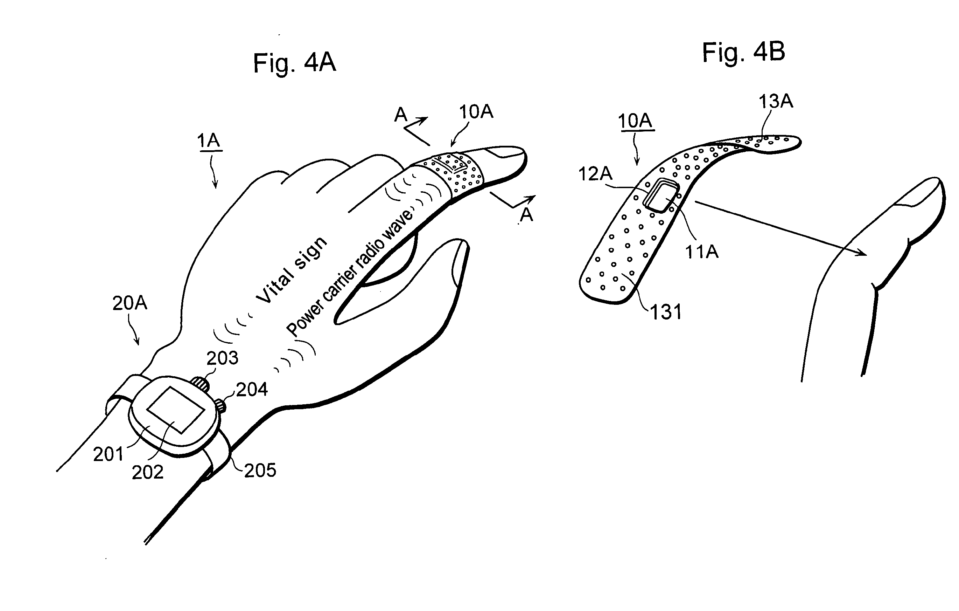

Figs.4A and 4B are diagrams showing an external view of an entire structure of the vital sign detection system 1A according to the first embodiment of the present invention. Of them, Fig.4A is a diagram showing the sensor device (also referred to as "vital sign detection sensor") 10A, which is a constituent element of the vital sign detection system 1A, being attached to a finger from which a vital sign is detected, as well as showing the sensor controlling device 20A being worn around a wrist of a user a little distant from the sensor device 10A, while Fig.4B is a diagram showing the sensor device 10A before being attached to the user.

As Figs.4A and 4B illustrate, the vital sign detection system 1A is comprised of the sensor device 10A that is attached to a finger or other detection positions and that detects such vital signs as blood flow, and of the sensor controlling device 20A that is attached to a wrist or other body parts and that transmits a power carrier radio wave used for supplying power to the sensor device 10A by radio and that receives a vital sign detected by the sensor device 10A by radio when supplying power to the sensor device 10A.

The sensor device 10A, which has an adhesive side 131, is comprised of an elongated oval-shaped tape 13A, an IC chip 11A that is placed approximately in the middle of the adhesive side 131 and that detects a vital sign, and an antenna coil 12A that takes energy from outside the device (in the present embodiment, a power carrier radio wave transmitted from the sensor controlling device 20A) and that transmits the vital sign detected by the IC chip 11A. Note that although the tape 13A has an elongated oval shape in the first embodiment, it is also possible to employ other shapes such as square and round shapes.

The sensor controlling device 20A is comprised of a wrist watch-shaped main body casing 201, a display 202 placed on the surface of the main body casing 201, a detection time setting button 203 placed on one side of the main body casing 201, an event button 204, a belt 205 and others.

The display 202, which is made up of an LCD (Liquid Crystal Display) and others, shows a result of data processing performed for a vital sign detected by the sensor device 10A (e.g. heart rate per unit of time, heart rate fluctuations).

The detection time setting button 203 is a button for setting in advance a time of day at which the detection of a vital sign is carried out. Detection times to be set by operating the detection time setting button 203 are dynamically determined according to the characteristics of a vital sign to be measured as well as to a physical condition of the user. To be more specific, when the user is in the resting condition, two detection times, one in the morning (8:00 a.m.) and the other in the evening (5:00 p.m.), for example, are set. When the user is ill, on the other hand, detection times are set for every hour, for example.

The event button 204 is a button to be operated when a measurement is performed of the user's will, if such an external factor as an instruction from a doctor makes the user to do so.

Note that the main body casing 201 includes a computer device that accepts detection times of a vital sign set by an operation on the detection time setting button 203 and a press of the event button 204 so as to cordlessly transmit a power carrier radio wave to the sensor device 10A at such detection times and when an event occurs; cordlessly receives a vital sign transmitted from the sensor device 10A when transmitting the power carrier radio wave; processes the received vital sign; and shows the result of such processing on the display 202.

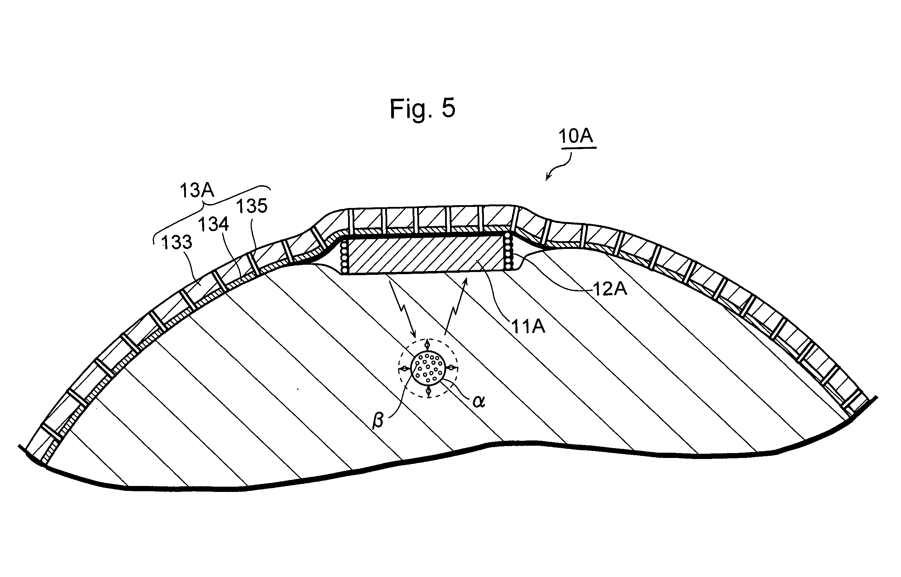

Fig.5 is a diagram showing a cross-sectional structure of the sensor device 10A when seen from the sectional line A-A shown in Fig.4A.

As illustrated in Fig.5, the sensor device 10A includes the IC chip 11A, the antenna coil 12A wound around the IC chip 11A, and the elongated oval-shaped tape 13A having the adhesive side 131.

The IC chip 11A is comprised of a rectifier diode that rectifies an electromotive force induced to the antenna coil 12A upon the receipt of the power carrier radio wave, a condenser that performs smoothing and accumulation of power, a light emitting diode and a photo diode that detect a vital sign, a transistor that performs analog processing and digital processing for the detected vital sign so as to transmit such vital sign via the antenna coil 12A and others, all of which are integrated within a few ∼ 10 square millimeters of a chip which is about 1 millimeter in thickness.

The tape 13A is made up of an elongated oval-shaped base material 133, an adhesive 134 that serves as the adhesive side 131 and a plurality of holes 135 passing through the base material 133 and the adhesive 134. The base material 133 is made of an elastic film material such as polyurethane and synthetic rubber which is about 1 millimeter in thickness. The adhesive 134, which is hydrophobic and has an anchoring effect, is made of hydrocolloid, acryl or rubber which is coated with the thickness of 0.1 millimeter.

With the sensor device 10A with the above configuration, by fixing the IC chip 11A and the antenna coil 12A on the tape 13A, having the surface of such IC chip 11A directly contact the user's skin on a vital sign detection position (e.g. finger), and adjusting the tension of the tape 13A being adhered around a finger, it is possible to make the IC chip 11A in intimate contact with the skin with an appropriate pressure.

Meanwhile, since the skin of a finger is thin, by applying to the internal skin of the finger a visible light (preferably, a blue light) from the light emitting diode of the IC chip 11A and receiving at the photo diode the visible light reflected inside the finger, it is possible to scan the interior of the finger. In so doing, it is witnessed that, as the heart beats, a blood-vessel α dilates and the amount of hemoglobin β in the blood vessel α increases with an increase in the amount of blood flow, and that the blood-vessel α contracts and the amount of hemoglobin β in the blood vessel α decreases with a decrease in the amount of blood flow. When the blood vessel α dilates, the amount of light received by the photo diode decreases due to light absorbing characteristics of the hemoglobin, while the amount of light received by the photo diode increases when the blood vessel α contracts. Accordingly, it is possible to detect variations in the amount of blood flow (vital sign) by checking the amount of reflection light received by the photo diode. Moreover, since the tape 13A firmly fixes the IC chip 11A onto the skin, preventing the misalignment of the IC chip 11A when the vital sign is detected, the reliability of the detected vital sign is increased.

Note that massive production makes it possible for the IC chip 11A to be manufactured at a very low cost. Therefore, when the tape 13A is getting dirty, the sensor device 10A whose tape 13A gets dirty is thrown away so as to be replaced with a new sensor device. Accordingly, the detection of a vital sign is always carried out in a hygienic state.

Moreover, although the antenna coil 12A is wound around the IC chip 11A in the present embodiment, it is also possible for such antenna coil 12A to be placed on the non-adhesive side of the tape 13A, with both edges of such antenna coil 12A penetrating the tape 13A so as to be connected to the IC chip 11A.

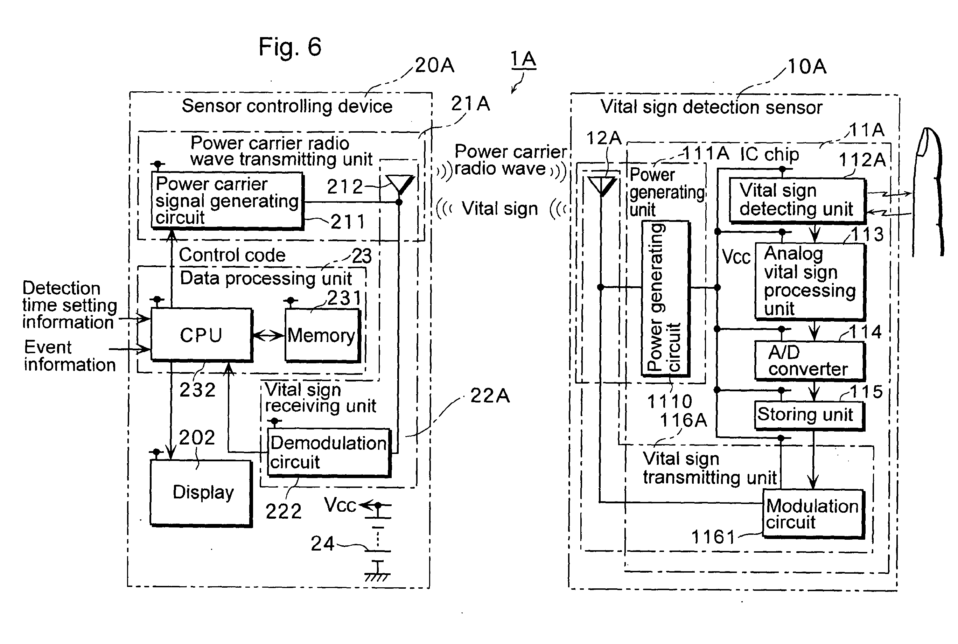

Fig.6 is a diagram showing the circuit configuration of the sensor device 10A (the IC chip11A and the antenna coil 12A) and the circuit configuration of the sensor controlling device 20A illustrated in Figs.4A and 4B.

In addition to the display 202, the sensor controlling device 20A comprises a power carrier radio wave transmitting unit 21A that transmits a power carrier radio wave to the sensor device 10A, a vital sign receiving unit 22A that receives a vital sign transmitted from the sensor device 10A by radio waves, a data processing unit 23 that controls the power carrier radio wave transmitting unit 21A to make it transmit the power carrier radio wave and stop transmitting the power carrier radio wave and that performs a variety of data processing on the basis of the vital sign received by the vital sign receiving unit 22A, and a battery 24 that supplies direct current power to the display 202, the power carrier radio wave transmitting unit 21A, the vital sign receiving unit 22A, and the data processing unit 23, and others.

The power carrier radio wave transmitting unit 21A includes a power carrier radio wave generating circuit 211 that generates a radio frequency (RF; e.g. 13.56MHz) power carrier signal while receiving a control code outputted from the data processing unit 23, and an antenna 212 that converts such power carrier signal generated by the power carrier radio wave generating circuit 211 into a power carrier radio wave and transmits the power carrier radio wave to the sensor device 10A.

The vital sign receiving unit 22A includes the antenna 212, which is shared with the power carrier radio wave transmitting unit 21A, for receiving the vital sign transmitted from the sensor device 10A by radio waves, and a demodulation circuit 222 that demodulates the vital sign received by the antenna 212.

The data processing unit 23 includes a memory 231 in which a vital sign and others received by the vital sign receiving unit 22A are stored, and the CPU 232 that incorporates a timer for timing and a ROM or the like for storing an application program in advance, that loads the application program stored in the ROM into the memory 231, and that has centralized control of the output of the control code transmitted to the power carrier radio wave generating circuit 211 and the termination of the output of the control code, on the basis of the application program loaded into the memory 231. More specifically, such CPU 232 executes the application program loaded into the memory 231; accepts detection time setting information which is set by an operation on the detection time setting button 203 and stores the detection time setting information in the memory 231; outputs the control code to the power carrier radio wave generating circuit 211 at the detection times stored in the memory 231; outputs the control code to the power carrier radio wave generating circuit 211 based on event information which is set by an operation on the event button 204; terminates the output of the control code to the power carrier radio wave generating circuit 211 after storing the vital sign received by the vital sign receiving unit 22A in the memory 231; and performs a variety of processing based on the vital sign stored in the memory 231 so as to show the result of such processing on the display 202.

In addition to the antenna coil 12A, the sensor device 10A comprises a power generating circuit 1110, a vital sign detecting unit 112A, an analog vital sign processing unit 113, an A/D converter 114, a storing unit 115, and a modulation circuit 1161, all of which are mounted on the IC chip 11A. Furthermore, the power generating circuit 1110 constitutes a power generating unit 111A together with the antenna coil 12A, and the modulation circuit 1161 constitutes a vital sign transmitting unit 116A together with the antenna coil 12A.

The antenna coil 12A of the power generating unit 111A receives the power carrier radio wave transmitted from the sensor controlling device 20A in compliance with either the electromagnetic induction method or the electromagnetic coupling method, and generates a high frequency electromotive force. The power generating circuit 1110 has a diode that rectifies such electromotive force and a condenser that smoothes the voltage of the rectified electromotive force to a certain value (Vcc) and that accumulates direct current power, and continuously supplies generated direct current power to the vital sign detecting unit 112A, the analog vital sign processing unit 113, the A/D converter 114, the storing unit 115, and the modulation circuit 1161 while the antenna coil 12A is receiving the power carrier radio wave.

The vital sign detecting unit 112A, which includes a light emitting diode and a photo diode, detects a vital sign by applying a blue light from the light emitting diode to the detection position upon the receipt of direct current power from the power generating unit 111A and by receiving at the photo diode a light reflected from the detection position. Then, specified processing is performed for such detected vital sign by the analog vital sign processing unit 113, the A/D converter 114, the storing unit 115, and the vital sign transmitting unit 116A in order of appearance, and the resulting vital sign is uploaded from the antenna coil 12A to the sensor controlling device 20A by radio.

To be more specific, when receiving direct current power from the power generating unit 111A, the analog vital sign processing unit 113 performs a variety of analog processing including the amplification of an analog detected signal, the removal of noise included in the detected signal, the filtering of the detected signal and the peak-hold of the detected signal in order to extract only necessary information from the vital sign detected by the vital sign detecting unit 112A, and then outputs the analog-processed vital sign.

The A/D converter 114, when receiving direct current power from the power generating unit 111A, samples the resulting vital sign at specified periods and converts the analog vital sign into a digital vital sign.

The storing unit 115, when receiving direct current power from the power generating unit 111A, temporally memorizes the digital vital sign.

The modulation circuit 1161 of the vital sign transmitting unit 116A, when receiving direct current power from the power generating unit 111A, reads the vital sign sequentially from the storing unit 115, and modulates a predetermined high frequency signal (e.g. 13.56MHz) by such readout vital sign. The antenna coil 12A converts the high frequency signal modulated by the vital sign into a radio wave, and transmits by radio waves the vital sign to the sensor controlling device 20A transmitting the power carrier radio wave. Note that, concerning a modulation method employed by the modulation circuit 1161, an arbitral method such as ASK (Amplitude Shift Keying) and FSK (Frequency Shift Keying) may be used as long as such a method corresponds to a demodulation method employed by the demodulation circuit 222.

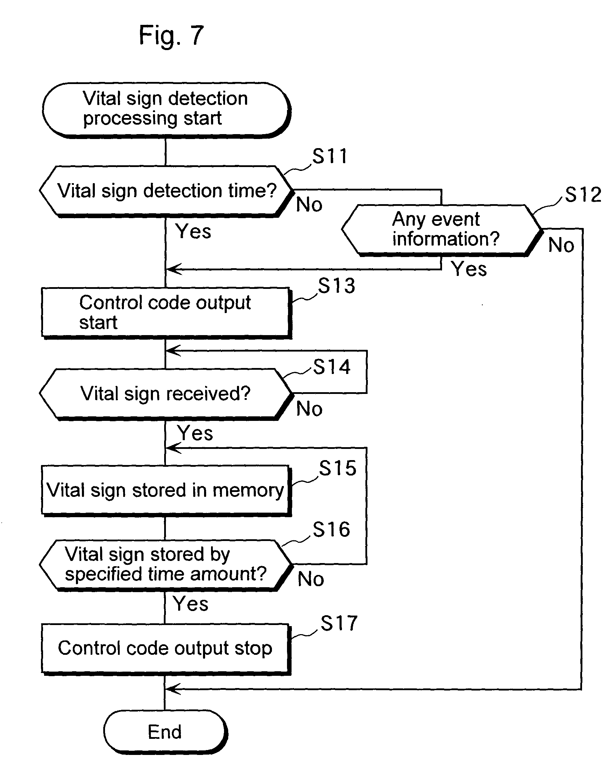

Next, referring to a flowchart in Fig.7, an explanation is given for the operation of the CPU 232 of the sensor controlling device 20A.

The CPU 232 executes the application program loaded into the memory 231 and judges whether it is a vital sign detection time or not (S11). Such judgment is made by comparing a time indicated by the internal timer with the detection time setting information stored in advance in the memory 231. If it is not a vital sign detection time (S11 "No"), the CPU 232 judges whether there is an input of event information or not (S12). If there is no input of event information (S12 "No"), the CPU 232 terminates the processing for vital sign detection.

In contrast, when it is a vital sign detection time in Step S11 (S11 "Yes"), or when there is an input of event information in Step S12, the CPU232 starts outputting the control code to the power carrier radio wave generating circuit 211 (S13), and waits for the vital sign to be transmitted from the demodulation circuit 222 (S14).

If the CPU 232 outputs the control code here, the power carrier radio wave transmitting unit 21 transmits the power carrier radio wave. The power generating unit 111A of the sensor device 10A which receives such power carrier radio wave, converts the power carrier radio wave into direct current power. Then, the power generating unit 111A supplies direct current power to the vital sign detecting unit 112A and other units so as to activate (energize) them. Consequently, the vital sign detecting unit 112A detects a vital sign, and such detected vital sign is transmitted being superimposed onto a radio wave, after being performed a variety of processing by the analog vital sign processing unit 113 and other units. The demodulation circuit 222 of the vital sign receiving unit 22A which receives such radio wave, demodulates the vital sign, and the demodulated vital sign is then inputted to the CPU 232. To put it another way, although no cord is used, it is possible to drive each unit including the vital sign detecting unit 112A and to perform the remote sensing of a vital sign, by having the power generating unit 111A of the sensor device 10A generate direct current power.

Upon the receipt of the vital sign, the CPU 232 stores the received vital sign in the memory 231 only by the amount of a specified time period (S15 and S16). In the case of taking a pulse from blood flow, a specified time period is 2∼3 seconds at minimum, a time period required to measure the interval between pulses, or preferably 1 minute.

After the vital sign by the amount of a specified time period is stored in the memory 231 (S16 "Yes"), the CPU 232 terminates the output of the control code to the power carrier radio wave generating circuit 211 (S17), and terminates the processing for vital sign detection. When the CPU 232 terminates the output of the control code, the power carrier radio wave transmitting unit 21 stops transmitting the power carrier radio wave. And when the transmission of the power carrier radio wave terminates, the power generating unit 111A of the sensor device 10A stops generating power so as to inactivate the vital sign detecting unit 112A and other units. Subsequently, the vital sign detecting unit 112A stops the detection of the vital sign, and the transmission of the vital sign by radio waves from the sensor device 10A to the vital sign receiving unit 22A of the sensor controlling device 20A is terminated. To put it another way, although no cord is used, it is possible to stop the operation of each unit including the vital sign detecting unit 112A and to stop the remote sensing of the vital sign, by having the power generating unit 111A of the sensor device 10A stop generating power.

By repeating the above processing, it is possible to perform the remote sensing of a vital sign at a detection time which is dynamically set according to a physical condition of the user as well as when an event occurs, i.e., a vital sign is measured of the user's will, due to an external factor (e.g. an instruction from a doctor).

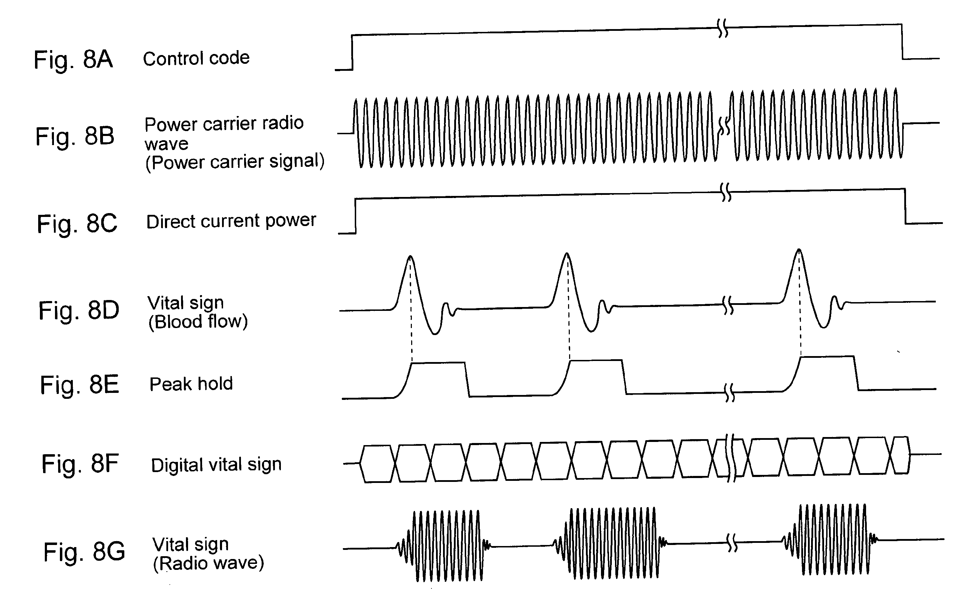

Next, referring to waveform charts shown in Figs.8A∼8G, an explanation is given for the operation that takes place at vital sign detection times.

As illustrated in Fig.8A, when a vital sign detection time comes and when there is an input of event information, the CPU 232 of the sensor controlling device 20A outputs the control code to the power carrier radio wave transmitting unit 21A. While the control code is being inputted, the power carrier radio wave transmitting unit 21A transmits the power carrier radio wave illustrated in Fig.8B to the sensor device 10A.

Upon the receipt of the power carrier radio wave, the antenna coil 12A that constitutes the power generating unit 111A of the sensor device 10A generates a power carrier signal that is a high frequency electromotive force through electromagnetic induction or electromagnetic coupling, while receiving such power carrier radio wave. The power generating circuit 1110 converts such power carrier signal into direct current power illustrated in Fig.8C by rectifying and smoothing the power carrier signal, and continuously supplies such direct current power to the vital sign detecting unit 112A, the analog vital sign processing unit 113, the A/D converter 114, the storing unit 115, and the modulation circuit 1161 of the vital sign transmitting unit 116A. Note that, since the frequency of the power carrier radio wave is high, only a very small capacity is required for the condenser employed in the power generating circuit 1110. Accordingly, it is possible for such condenser to be mounted on the chip.

When supplied with direct current power, the vital sign detecting unit 112A detects variations in blood flow of a user's blood vessel, i.e., an analog vital sign illustrated in Fig.8D, by applying a blue light to the detection position and then receiving a reflection light from the detection position, while direct current power is being supplied.

When supplied with direct current power, the analog vital sign processing unit 113, in order to extract necessary information (e.g. peak value of blood pressure) from the analog vital sign outputted from the vital sign detecting unit 112A, performs analog processing including noise removal, amplification, filtering and peak hold, and then outputs an analog vital sign illustrated in Fig.8E for which such analog processing is performed, while direct current power is being supplied.

When supplied with direct current power, the A/D converter 114 samples the analog vital sign outputted from the analog vital sign processing unit 113 so as to convert such analog vital sign into a digital vital sign (see Fig.8F), and outputs the converted vital sign, while direct current power is being supplied.

When supplied with direct current power, the storing unit 115 temporally memorizes the digital vital sign outputted from the A/D converter 114, while direct current power is being supplied.

When supplied with direct current power, the modulation circuit 1161 of the vital sign transmitting unit 116A reads the vital sign from the storing unit 115 and, by modulating the power carrier radio wave by such readout vital sign, uploads the vital sign to the sensor controlling device 20A via the antenna coil 12A by radio, while direct current power is being supplied (Fig.8G).

The CPU 232 of the sensor device 10A, after storing the uploaded vital sign by the amount of a specified time period in the memory 231, terminates the output of the control code, and the power carrier radio wave transmitting unit 21 stops transmitting the power carrier radio wave. Consequently, the power generating unit 111A of the sensor device 10A stops generating power, the vital sign detecting unit 112A terminates the detection of the vital sign, and the transmission of the vital sign by radio waves from the sensor device 10A to the vital sign receiving unit 22A of the sensor controlling device 20A is terminated.

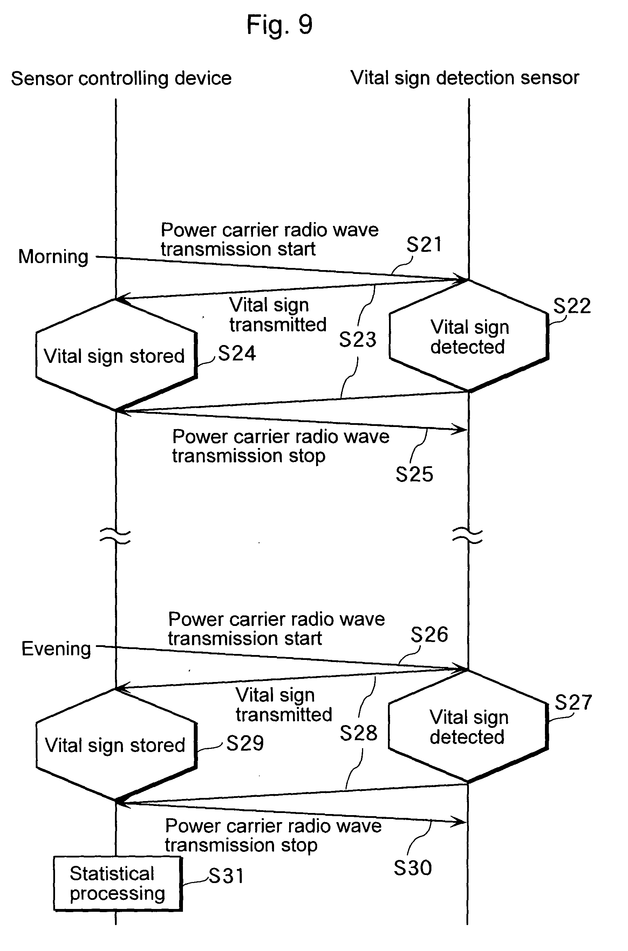

Fig.9 is a sequence diagram illustrating an exchange between the sensor controlling device 20A and the sensor device 10A. Note that this figure illustrates the case where two detection times, one in the morning and the other in the evening, are set.

When one detection time which is set in advance (e.g. 8:00 in the morning) comes, the CPU 232 of the sensor controlling device 20A starts outputting the control code to the power carrier radio wave generating circuit 211 of the power carrier radio wave transmitting unit 21A. Receiving the control code, the power carrier radio wave generating circuit 211 starts generating the power carrier signal. Subsequently, the antenna 212 starts transmitting the power carrier radio wave to the sensor device 10A (S21).

The antenna coil 12A of the sensor device 10A receives the power carrier radio wave, and the power generating circuit 1110 generates direct current power so as to supply generated direct current power to the vital sign detecting unit 112A ∼ the modulation circuit 1161 of the vital sign transmitting unit 116A. Subsequently, the vital sign detecting unit 112A detects a vital sign while being supplied with direct current power (S22). Then, while direct current power is being supplied, the modulation circuit 1161 of the vital sign transmitting unit 116A modulates the power carrier signal by the detected vital sign, and the antenna coil 12A sequentially transmits the vital sign by radio waves (S23).

When the vital sign receiving unit 22A of the sensor controlling device 20A receives through the antenna 212 the vital sign transmitted by radio, the demodulation circuit 222 demodulates such vital sign. Then, the CPU 232 of the sensor controlling device 20A has the memory 231 sequentially memorize the demodulated vital sign by the amount of a specified time period (S24). After the memory 231 fully memorizes the vital sign by the amount of a specified time period, the CPU 232 stops outputting the control code to the power carrier radio wave generating circuit 211. When the output of the control code stops, the power carrier radio wave generating circuit 211 stops generating the power carrier signal. Subsequently, the antenna 212 stops transmitting the power carrier radio wave (S25).

When the transmission of the power carrier radio wave is terminated, the power generating circuit 1110 stops the provision of direct current power. Consequently, the vital sign detecting unit 112A terminates the detection of the vital sign, and the vital sign transmitting unit 116A stops transmitting the vital sign.

Moreover, when t he other detection time which is set in advance (e.g. 5:00 in the evening) comes, the CPU 232 of the sensor controlling device 20A starts outputting the control code to the power carrier radio wave generating circuit 211 of the power carrier radio wave transmitting unit 21A. Subsequently, the antenna 212 starts transmitting the power carrier radio wave to the sensor device 10A (S26).

The power generating unit 111A of the sensor device 10A generates direct current power so as to supply generated direct current power to the vital sign detecting unit 112A ∼ the modulation circuit 1161 of the vital sign transmitting unit 116A. Subsequently, the vital sign detecting unit 112A detects a vital sign while being supplied with direct current power (S27). Then, while direct current power is being supplied, the modulation circuit 1161 of the vital sign transmitting unit 116A modulates the power carrier signal by the detected vital sign, and the antenna coil 12A sequentially transmits the vital sign by radio waves (S28).

When receiving the vital sign transmitted by radio, the vital sign receiving unit 22A of the sensor controlling device 20A demodulates such vital sign. Then, the CPU 232 of the sensor controlling device 20A has the memory 231 sequentially memorize the demodulated vital sign by the amount of a specified time period (S29). After the memory 231 fully memorizes the vital sign by the amount of a specified time period, the CPU 232 stops outputting the control code to the power carrier radio wave transmitting unit 21A. When the output of the control code is terminated, the power carrier radio wave transmitting unit 21A stops generating the power carrier signal. Subsequently, the antenna 212 stops transmitting the power carrier radio wave (S30).

When the transmission of the power carrier radio wave is terminated, the power generating circuit 1110 stops the provision of direct current power. Consequently, the vital sign detecting unit 112A terminates the detection of the vital sign, and the vital sign transmitting unit 116A stops transmitting the vital sign.

At the completion of both the morning and evening vital sign detections, the CPU 232 performs statistical processing on the basis of the vital sign stored in memory 231 by the amount of a specified time period in the morning and evening, and performs visualization processing which includes the presentation of a result of the statistical processing in graphical form (S31). More specifically, the CPU 232 searches the vital sign by the amount of a specified time period in the memory 231 so as to find each datum corresponding to the peak value of blood flow illustrated in Fig.8D, and then determines the interval between each peak value based on the number of samplings between them. Furthermore, the CPU 232 calculates a time average value between each peak value, divides the resulting average value by 60 seconds so as to determine a pulse rate per minute, and then shows the determined pulse rate on the display 202. Moreover, the CPU 232 also presents in graphical form fluctuations with respect to the average values between each peak value.

As a result, it is possible for the sensor controlling device 20A to perform the remote sensing of a vital sign by remotely activating the power generating unit 111A of the sensor device 10A at the preset detection times (8:00 in the morning and 5:00 in the evening) so as to make it generate power and supply generated power to the vital sign detecting unit 112A and other units.

Note that the present embodiment describes the case where detection times are set one in the morning and the other in the evening, but if detection times are set for every hour, it is possible to perform the remote sensing of a vital sign on an hourly basis. Furthermore, it is also possible to remotely sense a vital sign, when an even occurs, that is, when a vital sign is measured of the user's will due to such an external factor as an instruction from a doctor.

Moreover, although statistical processing is performed only after the evening detection in the present embodiment, it is also possible to carry out statistical processing each time the remote sensing of a vital sign is performed.

As described above, the sensor device 10A according to the first embodiment does not include any battery for accumulating charge as the foregoing prior arts have, but includes the power generating unit 111A instead that generates power in accordance with the electromagnetic induction method or the electromagnetic coupling method. Accordingly, as compared with a sensor device which requires a battery, the present invention makes it possible to make a significant reduction in the size of a sensor device. Furthermore, since there is no need for battery changes, the operating cost of the sensor device 10A will be much lowered. More importantly, since the power generating circuit 1110 can be integrated onto an LSI, it is possible to make a significant reduction in the size as well as in the manufacturing cost of a sensor device by integrating all the circuits excluding the antenna coil 12A (i.e. the power generating circuit 1110 and the vital sign detecting unit 112A ∼ the modulation circuit 1161 of the vital sign transmitting unit 116A) onto an LSI as a single chip.

Meanwhile, unlike a power supply method utilizing a cable, the sensor device 10A according to the present embodiment employs no cord (wire), meaning that there is no concern that the sensor device 10A will be misaligned or coming off because of a dragged cable, as in the case of the prior arts. Therefore, it is obvious that such sensor device 10A will eliminate the user's inconveniences that would be caused by a cable when wearing the sensor device.

As is obvious, since the absence of a cable requires no manufacturing step for connecting a cable to the sensor device 10A or to the sensor controlling device 20A, and the production of the sensor device 10A according to the present embodiment is simple enough, it is possible to lower the manufacturing cost through the reduction in the number of manufacturing steps.

The present invention is characterized by that it makes it possible for the sensor device 10A, which is required to be fixed firmly onto the user, to be wirelessly connected to the sensor controlling device 20A, which is preferably attached to the user loosely, by completely separating the sensor device 10A from the sensor controlling device 20A, and that the sensor device 10A is greatly reduced in size with a configuration that does not include any battery.

Accordingly, it is possible to reduce the size of the sensor device 10A and to improve the reliability of data acquired by the sensor device 10A because the sensor device 10A will not provide incorrect data by being misaligned or coming loose from a specified position.

Furthermore, the sensor device 10A will be available at a very inexpensive price through the reduction in the number of its elements made possible by the power generating unit 111A that generates power and therefore eliminates the need for a battery as well as in the number of manufacturing steps made possible by the absence of a cable, and through integrating circuits on a chip. Moreover, if the price is lowered, it is possible for the sensor device 10A, which directly contacts the user's skin, to be made disposable, realizing the vital sign detection system 1A in which a vital sign is detected in a highly safe and hygienic manner.

### (The Second Embodiment)

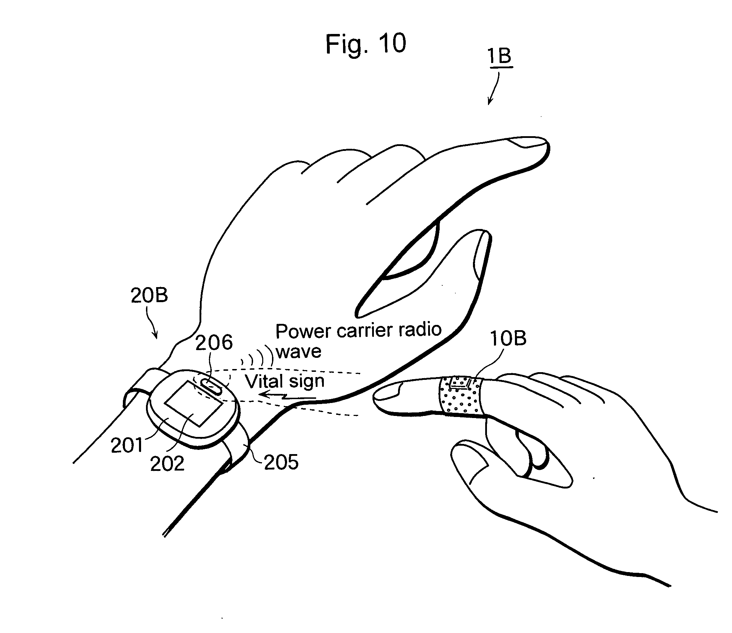

Fig.10 is a diagram showing an external view of an entire structure of the vital sign detection system 1B according to the second embodiment of the present invention. The diagram illustrates the sensor device 10B, which is a constituent element of the vital sign detection system 1B, being attached to a right finger, as well as the sensor controlling device 20B being worn around the left wrist. Note that elements corresponding to those in the vital sign detection system 1A are assigned the same numbers as those used in the first embodiment of the present invention, and therefore that detailed explanations thereof are omitted, with explanations given only for elements different from the vital sign detection system 1A.

As illustrated in Fig.10, the vital sign detection system 1B is identical to the first embodiment in that it is comprised of the sensor device 10B and the sensor controlling device 20B. However, the present embodiment is different from the first embodiment in that the sensor controlling device 20B includes a touch sensor 206 instead of the detection time setting button 203 and the event button 204 of the sensor controlling device 20A, and a power carrier radio wave is transmitted while such touch sensor 206 is being pressed down. Also, the vital sign detection system 1B of the second embodiment is different from the vital sign detection system 1A of the first embodiment in that a human body (a finger tip, in the present embodiment) between the sensor device 10B and the touch sensor 206 of the sensor controlling device 20B serves as a signal transmission line (so-called "body LAN"), and a vital sign is transmitted from the sensor device 10B to the sensor controlling device 20B via such body LAN.

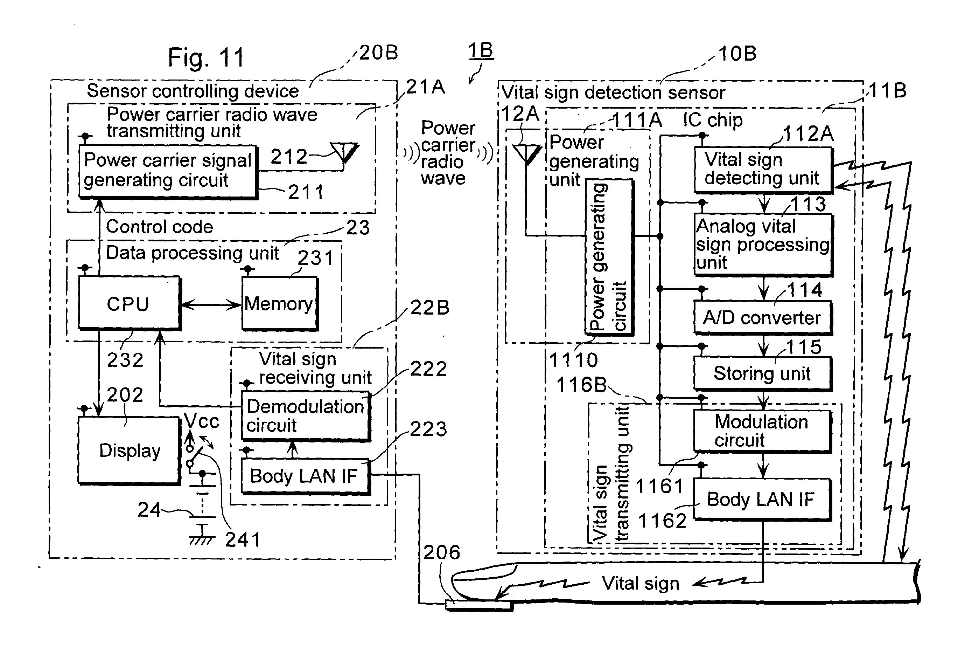

Fig.11 is a diagram showing the circuit configurations of the sensor device 10B and the sensor controlling device 20B illustrated in Fig.10. Note that elements that correspond to those in the sensor device 10A and the sensor controlling device 20A illustrated in Fig.6 are assigned the same numbers and therefore that detailed explanations thereof are omitted with explanations given only for different elements.

The sensor controlling device 20B is approximately the same configuration as the sensor controlling device 20A illustrated in Fig.6, but it is different from the sensor controlling device 20A in that a vital sign receiving unit 22B made up of a body LAN interface 223 and the demodulation circuit 222 is employed and that a relay switch 241 of a latching relay, placed in the direct current power supply channel leading from the battery 24 to each circuit, which operates synchronously with a press operation on the touch sensor 206.

Note that, even after a press operation on the touch sensor 206 is released, the relay switch 241 maintains continuity until the CPU 232 deactivates a relay coil of the latching relay.

The body LAN interface 223, which is comprised of electrodes and others serving as an electrostatic capacity in between the human body, receives via the touch sensor 206 a high frequency electric signal superimposed with a vital sign transmitted via the human body. The demodulation circuit 222 modulates the vital sign superimposed onto the high frequency electric signal outputted from the body LAN interface 223, and outputs the modulated vital sign to the CPU 232.

The sensor device 10B is approximately the same configuration as the sensor device 10A illustrated in Fig.6, but it is different from the sensor device 10A in that a vital sign transmitting unit 116B, which is placed inside an IC chip 11B and made up of the modulation circuit 1161 and a body LAN interface 1162, is employed instead of the vital sign transmitting unit 116A.

The body LAN interface 1162, as in the case of the body LAN interface 223, is comprised of electrodes and others serving as an electrostatic capacity in between the human body, and transmits via the human body the high frequency electric signal superimposed with the vital sign outputted from the modulation circuit 1161.

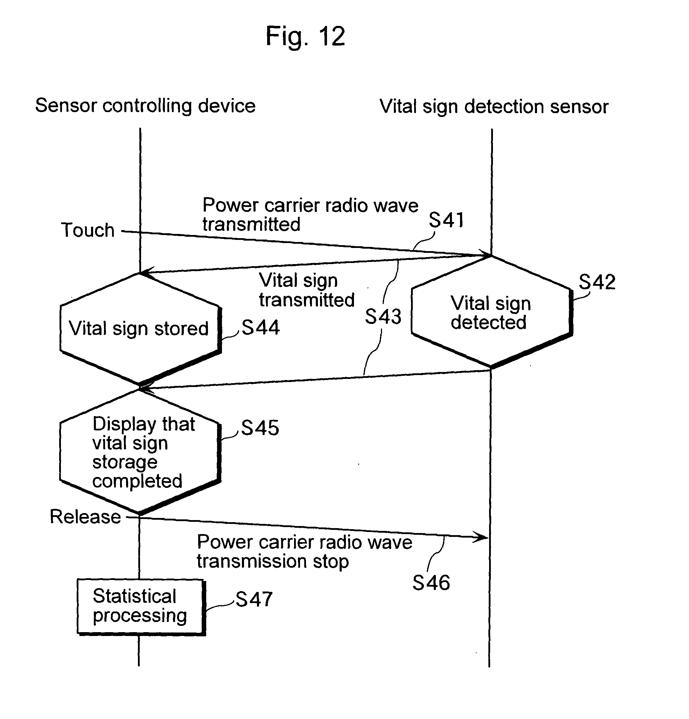

Referring to a sequence diagram shown in Fig.12, an explanation is given for an exchange between the sensor device 10B and the sensor controlling device 20B with the above configuration.

First, when a user wearing the sensor device 10B touches the touch sensor 206 of the sensor controlling device 20B, the relay switch 241 starts conducting in response to the press operation on the touch sensor 206, and direct current power from the battery 24 is supplied to the power carrier radio wave generating unit 211, the memory 231, the CPU 232, the demodulation circuit 222, the body LAN interface 223, and the display 202 via the relay switch 206. When supplied with direct current power, the CPU 232 loads an application program stored in a ROM into the memory 231 and then outputs the control code to the power carrier radio wave transmitting unit 21A. Subsequently, the power carrier radio wave transmitting unit 21A starts transmitting the power carrier radio wave to the sensor device 10B (S41). Note that when outputting the control code, the CPU 232 drives the relay coil of the latching relay at the same time so as to maintain continuity of the relay switch 241.

The power generating unit 111A of the sensor device 10B generates direct current power by receiving the power carrier radio wave, and supplies generated direct current power to the vital sign detecting unit 112A ∼ the vital sign transmitting unit 116B. Consequently, the vital sign detecting unit 112A detects a vital sign while being supplied with direct current power (S42). Meanwhile, while being supplied with direct current power, the modulation circuit 1161 of the vital sign transmitting unit 116B modulates the power carrier signal by the detected vital sign, and the body LAN interface 1162 sequentially transmits an electric signal superimposed with the vital sign to the human body (S43).

The body LAN interface 223 of the sensor controlling device 20B receives the electric signal superimposed with the vital sign transmitted via the touch sensor 206 and the human body, and the demodulation circuit 222 demodulates the vital sign included in the high frequency signal outputted from the body LAN interface 223. The CPU 232 of the sensor controlling device 20B has the memory 231 sequentially memorize the demodulated vital sign by the amount of a specified time period (S44). Then, after the memory 231 fully memorizes the vital sign by the amount of a specified time period, the CPU 232 shows a message on the display 202 indicating that the vital sign is stored and asking the user to release the touch sensor 206 (S45). On release of the touch sensor 206, the CPU 232 stops outputting the control code to the power carrier radio wave transmitting unit 21A. Then, when the output of the control code is terminated, the power carrier radio wave transmitting unit 21A stops transmitting the power carrier radio wave (S46).

When the transmission of the power carrier radio wave stops, the power generating circuit 1110 of the sensor device 10B stops supplying direct current power. Consequently, the vital sign detecting unit 112A terminates the detection of the vital sign, and the vital sign transmitting unit 116A stops transmitting the vital sign.

After terminating the output of the control code, the CPU 232 performs statistical processing on the basis of the vital sign stored in the memory 231 by the amount of a specified time, and shows a result of the statistical processing onto the display 202 for a specified period of time (S47). Then, after such specified period of time, the CPU 232 terminates the operation of the relay coil of the latching relay so as to trip the relay switch 241.

Note that although the transmission of the power carrier radio wave is terminated after the touch sensor 206 is released in the above sequences, it is also possible to terminate the transmission of the power carrier radio wave immediately after the vital sign is stored in the memory by the amount of a specified time period, and then to show onto the display 202 a message indicating that the storage of the vital sign completes and asking the user to release the touch sensor.

As described above, according to the sensor device 10B presented in the second embodiment, it is possible to provide a vital sign detection sensor that is small in size and capable of reliably detecting a vital sign, as well as a sensor controlling device that controls such sensor, as in the case of the sensor device 10A according to the first embodiment. Furthermore, it is also possible to provide a disposable vital sign detection sensor and a sensor controlling device that controls such sensor.

Moreover, with the sensor controlling device 20B according to the present embodiment, since the application program dynamically changes the frequency of obtaining a vital sign depending on a physical condition of the user, the characteristics of the vital sign and external factors, and the battery 24 of the sensor controlling device 20B is used only in such cases, it is possible to reduce to a minimum the amount of power consumed by the sensor controlling device 20B. This results in a small number of times for which a battery is changed for the sensor controlling device 20B, leading to the reduction in the operating cost of the sensor controlling device 20B.

### (The Third Embodiment)

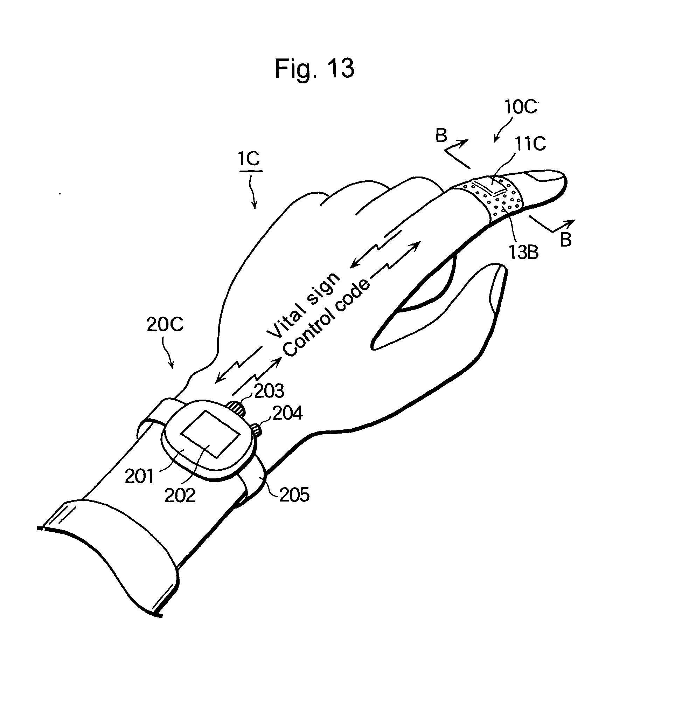

Fig.13 is a diagram showing an external view of an entire structure of the vital sign detection system 1C according to the third embodiment of the present invention. The diagram illustrates the sensor device 10C, which is a constituent element of the vital sign detection system 1C, being attached to a left finger, as well as the sensor controlling device 20C being worn around the left wrist. Note that elements corresponding to those in the vital sign detection system 1A are assigned the same numbers as those used in the first embodiment of the present invention, and therefore that detailed explanations thereof are omitted, with explanations given only for elements different from the vital sign detection system 1A.

As illustrated in Fig.13, the vital sign detection system 1C is identical to the first embodiment in that it is comprised of the sensor device 10C and the sensor controlling device 20C. However, the present embodiment is different from the sensor device 10A, which generates power depending on the power carrier radio wave transmitted from the sensor controlling device 20A, in that the sensor device 10C generates power independently of the sensor controlling device 20C through a self-contained battery so-called solar battery that takes energy (light) from outside the device so as to convert such light energy into direct current power.

Meanwhile, in this sensor device 10C, since direct current power from such solar battery is constantly supplied to units including a vital sign detecting unit, it is also possible to operate the vital sign detecting unit and other units all the time so as to continuously detect a vital sign. However, the amount of power a few times larger than that of when no detection of a vital sign is carried out, i.e., at sleep mode, is consumed by the vital sign detecting unit and other units for detecting a vital sign. This consequently necessitates the amount of power generated by the solar battery to be increased by the amount of such increase. Accordingly, it is preferable to have the vital sign detecting unit and other units stay in sleep mode under normal conditions and to activate and have them detect a vital sign only when necessary. Therefore, the present embodiment is different from the first embodiment in that the sensor controlling device 20C transmits the control code by lightwaves when necessary and the sensor device 10C continuously detects a vital sign only while receiving the control code.

Furthermore, the third embodiment is also different from the first embodiment, in which a vital sign is transmitted/received by radio waves utilizing the antenna, in that the sensor device 10C is configured to transmit the detected vital sign by lightwaves, and the sensor controlling device 20C is configured to receive the vital sign by lightwaves without having any antenna.

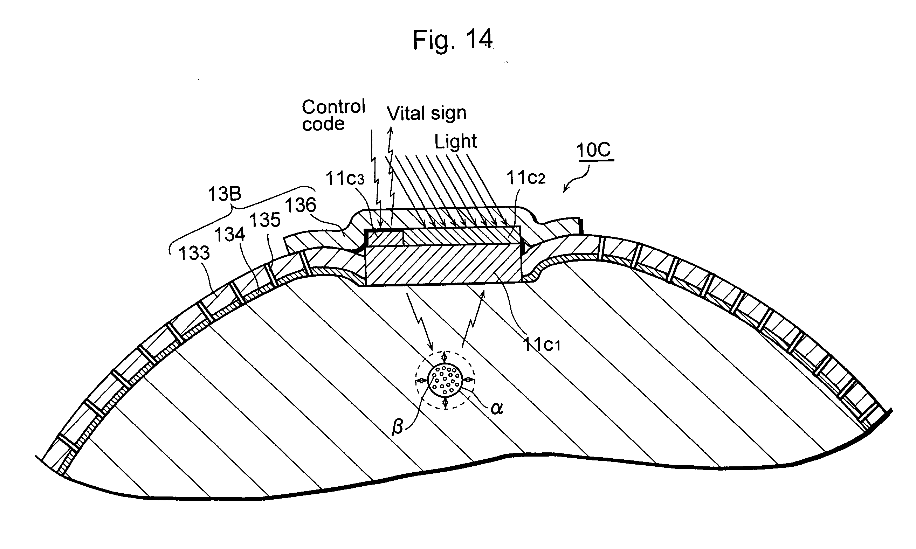

Fig.14 is a diagram showing a cross-sectional structure of the sensor device 10C when seen from the sectional line B-B shown in Fig.13.

As illustrated in Fig.14, the sensor device 10C is comprised of an IC chip11C and a tape 13B.

The IC chip 11C is made up of an IC chip 11c1 with approximately the same configuration as that of the IC chip11A, a solar battery 11c2 to be adhered to the IC chip 11c1, and a light communication unit 11c3 to be adhered to the IC chip 11c1.

The solar battery 11c2 takes in energy (light) externally and converts it into direct current power.

The light communication unit 11c3 carries out a light communication with the sensor controlling device 20C so as to receive light modulated by the control code and to transmit a vital sign detected by the IC chip 11c1 by lightwaves.

In addition to the base material 133 and the adhesive 134, the tape 13B includes a transparent sheet 136 to be adhered over the surface of the base material 133, and is configured not to interfere with the reception of light by the solar battery 11c2 or the light communication of the light communication unit 11c3.

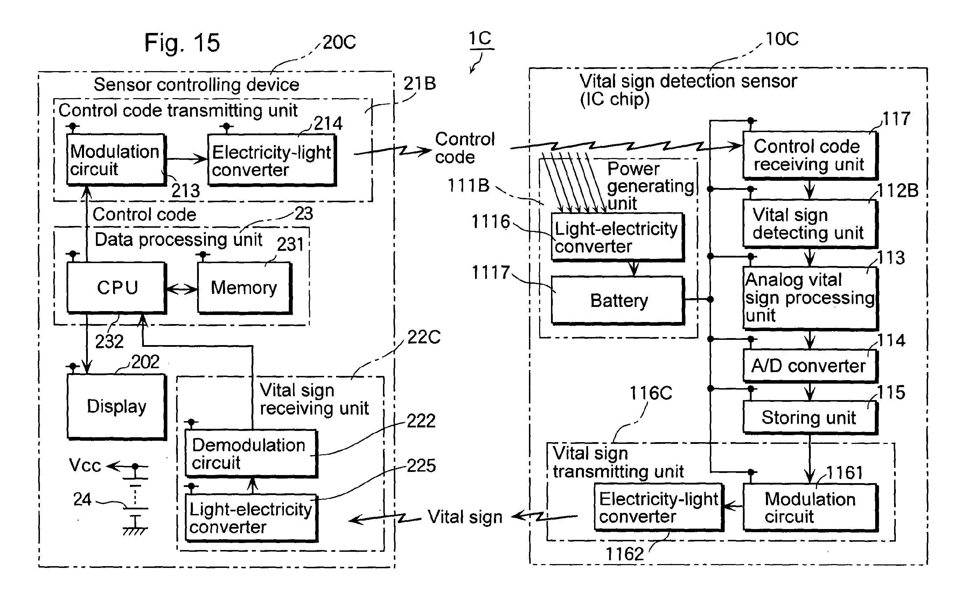

Fig.15 is a diagram showing the circuit configurations of the sensor device 10C and the sensor controlling device 20C illustrated in Fig.13. Note that elements that correspond to those in the sensor device 10A and the sensor controlling device 20A illustrated in Fig.6 are assigned the same numbers and therefore that detailed explanations thereof are omitted with explanations given only for different elements.

A control code transmitting unit 21B of the sensor controlling device 20C is comprised of a modulation circuit 213 that modulates a high frequency signal by the control code and an electricity-light converter 214 that converts the high frequency electric signal superimposed with the control code into light and transmits the light superimposed with the control code to the sensor device 10C.

A vital sign receiving unit 22C is comprised of a light-electricity converter 225 that receives the vital sign transmitted by lightwaves from the sensor device 10C and converts such vital sign into a high-frequency electric signal superimposed with the vital sign, and the demodulation circuit 222 that demodulates the vital sign included in the high-frequency electric signal.

A power generating unit 111B of the sensor device 10C is made up of a light-electricity converter 1116 and a battery 1117. The light-electricity converter 1116, which is made of amorphous or the like, is capable of extracting electric energy (direct current power) in a highly efficient manner from outside light coming into a room or such indoor light as fluorescent light. The battery 1117, which includes a condenser and others, accumulates direct current power generated by the light-electricity converter 1116.

A control code receiving unit 117 includes: a light-electricity converting unit that receives the light superimposed with the control code transmitted from the sensor controlling device 20C and converts such light into a high-frequency electric signal superimposed with the control code; a demodulator that demodulates the control code included in the high-frequency electric signal and keeps a vital sign detecting unit 112B activated while the control code is being demodulated; and others.

The vital sign detecting unit 112B is configured to detect a vital sign when supplied with direct current power from the power generating unit 111B and when activated by the control code receiving unit 117.

A vital sign transmitting unit 116C is made up of a modulation circuit 1161 and an electricity-light converter 1165, and transmits a vital sign by lightwaves in accordance with such a communication protocol as IrDA which is a standard protocol for PC communications.

Meanwhile, direct current power from the battery 1117 of the power generating unit 111B is supplied to units including the vital sign detecting unit 112B, but the vital sign detecting unit 112B and other units are in sleep mode when the detection of a vital sign is not carried out. Therefore, the amount of power consumed by the vital sign detecting unit 112B in sleep mode is extremely small. As a result, it is not necessary for the battery 1117 to store such an amount of power as can drive the sensor device 10C for a long period of time, and the amount of power with which the sensor device 10C can be operated while no more than one cycle of sequences from the detection of a vital sign through to the transmission of the vital sign is carried out, i.e. a few seconds to a minute at the longest, is just required to be stored in the battery 1117. More importantly, direct current power generated by the light-electricity converter 1116 is replenished to the battery 1117 during such sequences, and therefore only a small condenser is required for the battery 1117, meaning that such a small condenser can be mounted on an IC chip.

Note that the vital sign detecting unit 112B, the analog vital sign processing unit 113, the A/D converter 114 and the modulation circuit 1161 are mounted on the IC chip 11c1 illustrated in Fig.14, the power generating unit 111B on the solar battery 11c2 illustrated in Fig.14, and the control code receiving unit 117 and the electricity-light converter 1165 on the light communication unit 11c3 illustrated in Fig.14.

Next, an explanation is given for an exchange carried out between the sensor controlling device 20C and the sensor device 10C in the vital sign detection system 1C with the above configuration. Note that such exchange is explained with reference to Fig.9 which illustrates the exchange between the sensor controlling device 20A and the sensor device 10A in the vital sign detection system 1A, because these two systems have the same processing procedure and their difference is that the transmission of the power carrier radio wave is changed to the transmission of the control code and the transmission of a vital sign by radio waves is changed to the transmission of a vital sign by lightwaves.

When one detection time which is set in advance (e.g. 8:00 in the morning) comes, the CPU 232 of the sensor controlling device 20C starts outputting the control code to the modulation circuit 213 of the control code transmitting unit 21B. After receiving the control code, the modulation circuit 213 starts generating the high-frequency signal superimposed with the control code. Subsequently, the electricity-light converter 214 starts transmitting light superimposed with the control code to the sensor device 10C (S21).

A power generating unit 111C of the sensor device 10C converts the received light into electric energy in the light-electricity converter 1116 and recharges the battery 1117, and the battery 1117 supplies direct current power to each unit including the control code receiving unit 117 and the vital sign detecting unit 112B. While receiving the light superimposed with the control code and the control code remains superimposed, the control code receiving unit 117 keeps the vital sign detecting unit 112B activated. Subsequently, the vital sign detecting unit 112B detects a vital sign while being active (S22). Then, while the vital sign detecting unit 112B is active, the modulation circuit 1161 of the vital sign transmitting unit 116C modulates the high-frequency signal by the detected vital sign, and the electricity-light converter 1165 sequentially transmits the vital sign by lightwaves (S23).

The vital sign receiving unit 22C of the sensor controlling device 20C converts in the light-electricity converter 225 the vital sign transmitted by lightwaves into a high-frequency signal superimposed with the vital sign, and the demodulation circuit 222 demodulates such vital sign included in the high-frequency signal. Then, the CPU 232 has the memory 231 sequentially memorize the demodulated vital sign by the amount of a specified time period (S24). After the memory 231 fully memorizes the vital sign by the amount of a specified time period, the CPU 232 stops outputting the control code to the control code transmitting unit 21B. When the output of the control code is terminated, the control code transmitting unit 21B stops generating the control code. And when the output of the control code is terminated, the control code transmitting unit 21B stops the transmission of the control code by lightwaves (S25).

When the transmission of the control code by lightwaves is terminated, the control code receiving unit 117 of the sensor device 10C deactivates the vital sign detecting unit 112B. Consequently, the vital sign detecting unit 112B terminates the detection of the vital sign, the vital sign transmitting unit 116C stops transmitting the vital sign, and the vital sign detecting unit 112B and other units enter sleep mode.

Just like the case of the morning detection, when the evening detection time comes, the vital sign detection starts when the transmission of the control code by lightwaves starts, and after the memory 231 memorizes the vital sign by the amount of a specified time period, the detection of the vital sign stops at the termination of control code transmission by lightwaves.

At the completion of both the morning and evening vital sign detections, the CPU 232 performs statistical processing on the basis of the vital sign stored in memory 231 by the amount of a specified time period in the morning and evening, and performs visualization processing which includes the presentation of a result of the statistical processing in graphical form (S31).

As described above, it is possible for the sensor device 10C according to the third embodiment to provide a vital sign detection sensor that is small in size and capable of reliably detecting a vital sign, as well as a sensor controlling device that controls such sensor, as in the case of the sensor devices 10A and 10B according to the first and the second embodiments. Furthermore, it is also possible to provide a disposable vital sign detection sensor and a sensor controlling device that controls such sensor.

Furthermore, since the sensor device 10C does not require any antenna, meaning that there exists no step for mounting an antenna onto the sensor device 10C, it is possible to realize a configuration of the sensor device 10C which can be provided at a further inexpensive price. Moreover, since the sensor device 10C comprises the solar battery (the power generating unit 111B), it does not require power provision from the sensor controlling device 20C. This consequently reduces to a minimum the amount of power consumed by the sensor controlling device 20C and results in a small number of times for which a battery is changed for the sensor controlling device 20C, leading to the reduction in the operating cost of the sensor controlling device 20C.

Moreover, although the antenna coil 12A is used as an antenna for receiving the power carrier radio wave and as an antenna for transmitting a vital sign in the sensor devices 10A and 10B according to the first and the second embodiments, a different antenna may be employed for each purpose.

What is more, although the antenna coil 12A is wound around the IC chip 11A, it is also possible to place such antenna coil 12A on the surface of the tape.

Also, the storing unit 115 of the sensor devices 10A, 10B and 10C of the first to the third embodiments may be configured as a volatile memory or as a nonvolatile memory. When configured as a nonvolatile memory, since vital sign data is retained as it is even if power supply stops before the transmission of the vital sign (before the vital sign is fully transmitted). Therefore, when power is supplied next time, it is possible to transmit such previous data again, which consequently results in improved reliability of data detected by the sensor device 10A. Furthermore, it is also possible to omit the storing unit 115, and a digital vital sign converted by the A/D converter 114 is directly inputted to the vital sign transmitting unit 116A and then transmitted.

Moreover, although the sensor controlling devices 20A, 20B and 20C of the first to the third embodiments take a wrist watch form, they are not restricted to the form of a wrist watch to be fixed onto an arm, and therefore a sensor controlling device may take the form of a necklace to be dangled around the neck with a cord, or may be configured as something that can be put in a user's bag, for example. To put it another way, a sensor controlling device may take any form as long as it can be separated from a sensor device and placed in a place within which the sensor device can receive a power carrier radio wave and a control code.

Furthermore, although the third embodiment is configured to control how a vital sign starts to be obtained in the sensor device 10C by using the control code, the sensor controlling device 20C may be configured not to include the power carrier radio wave transmitting unit 22B and the sensor device 10C may be configured not to include the control code receiving unit 117. In this case, the transmission of a vital sign is carried out all the time with the sensor device 10C as a master, and a role of the sensor controlling device 20C is only to process the vital sign transmitted from the sensor device 10C as a slave.

Also, although the sensor devices 10A, 10B and 10C are attached to the surface of a human body with the tapes 13A and 13B in the first to the third embodiments, it is possible that such sensor devices 10A, 10B and 10C are implanted into a human body. In such case, there is no need for changing the tapes 13A and 13B, allowing a perpetual use of the sensor devices 10A, 10B and 10C.

Furthermore, although the sensor devices 10A, 10B and 10C in the first to the third embodiments are attached to a human body, such sensor devices may also be attached to animals such as a dog, a cat, a bird and a whale so as to obtain their vital signs. In this case, it is preferable that a position detection device including a GPS device is also attached to an animal together with a sensor device. Accordingly, the acquisition of detailed information on animal ecology embracing vital signs and position information is realized.

## Claims

1. A vital sign detection sensor that is portable and used attached to a human body, comprising:
a power generating unit operable to take in energy externally and generate direct current power from the energy;
a vital sign detecting unit operable detect a vital sign of a user while being supplied with the direct current power from the power generating unit; and
a vital sign transmitting unit operable to transmit the detected vital sign cordlessly while being supplied with the direct current power from the power generating unit.

2. The vital sign detection sensor according to Claim1,
wherein the energy is a power carrier radio wave, and
the power generating unit is equipped with an antenna that receives the power carrier radio wave.

3. The vital sign detection sensor according to Claim1,
wherein the energy is light energy, and
the power generating unit includes a light-electricity converting unit operable to convert the light energy into electric energy.

4. The vital sign detection sensor according to Claim1,
wherein the vital sign generating unit is equipped with an antenna that transmits the vital sign by radio.

5. The vital sign detection sensor according to Claim1,
wherein the vital sign generating unit includes an interface for transmitting the vital sign by using a body of the user as a transmission medium.

6. The vital sign detection sensor according to Claim1,
wherein the vital sign generating unit includes an electricity-light converting unit operable to transmit the vital sign by lightwaves.

7. The vital sign detection sensor according to Claim1,
wherein the vital sign detecting unit and at least a part of the power generating unit and the vital sign transmitting unit are integrated into one or more chips.

8. The vital sign detection sensor according to Claim7 further comprising a tape that has an adhesive side on which said one or more chips are attached and that is elastic,
wherein said one or more chips are firmly fixed on a detection position of the user's body by winding the tape around the detection position, with a vital sign detecting side of said one or more chips contacting the detection position.

9. The vital sign detection sensor according to Claim8,
wherein at least one of the power generating unit and the vital sign transmitting unit includes an antenna used for one of the following purposes: receiving the power carrier radio wave and transmitting the vital sign, and
the antenna is placed in one of the following positions: around said one or more chips and on a non-adhesive side of the tape.

10. A sensor controlling device that receives a vital sign transmitted from a vital sign detection sensor and controls a vital sign detection operation of the vital sign detection sensor, the sensor controlling device comprising:
a power carrier radio wave transmitting unit operable to transmit a power carrier radio wave when the vital sign detection sensor detects the vital sign;
a vital sign receiving unit operable to cordlessly receive the vital sign transmitted from the vital sign detection sensor;
a memory unit operable to memorize the vital sign received by the vital sign receiving unit by an amount of a specified time period; and
a controlling unit operable to control the power carrier radio wave transmitting unit transmitting the power carrier radio wave, from when the vital sign detection operation of the vital sign detection sensor starts to when the memory unit finishes memorizing the vital sign by the amount of the specified time period.

11. The sensor controlling device according to Claim10,
wherein the vital sign is transmitted from the vital sign detection sensor by radio, and
the vital sign receiving unit is equipped with an antenna that receives the vital sign transmitted from the vital sign detection sensor by radio.

12. The sensor controlling device according to Claim10,
wherein the vital sign is transmitted from the vital sign detection sensor by lightwaves, and
the vital sign receiving unit includes a light-electricity converting unit operable to receive the vital sign transmitted from the vital sign detection sensor by lightwaves.

13. The sensor controlling device according to Claim10,
wherein the controlling unit has the power carrier radio wave transmitting unit start transmitting the power carrier radio wave at a predetermined time.

14. The sensor controlling device according to Claim10,
wherein the controlling unit has the power carrier radio wave transmitting unit start transmitting the power carrier radio wave when an event occurs.

15. The sensor controlling device according to Claim10 further comprising a touch sensor that a user wearing the vital sign detection sensor touches,
wherein the controlling unit has the power carrier radio wave transmitting unit start transmitting the power carrier radio wave when the user wearing the vital sign detection sensor touches the touch sensor.

16. The sensor controlling device according to Claim15 further comprising:
a battery for supplying power to device components; and
a power supply switching unit operable to connect a power supply channel between the battery and the device components, when the user wearing the vital sign detection sensor touches the touch sensor, synchronously with the touch.

17. The sensor controlling device according to Claim15,
wherein the vital sign is transmitted via the user's body as a transmission medium, and
the vital sign receiving unit includes an interfacing unit operable to receive via the touch sensor the vital sign transmitted from the vital sign detection sensor using the user's body as the transmission medium.

18. The sensor controlling device according to Claim10,
wherein the controlling unit performs statistical processing for the vital sign stored in the memory unit by the amount of the specified time period, and determines the number of pulses per unit of time.

19. The sensor controlling device according to Claim18 further comprising a display unit,
wherein the controlling unit has the display unit display the number of the pulses per unit of time determined by the statistical processing.

20. The sensor controlling device according to Claim18,
wherein the controlling unit further determines period variations of the number of the pulses.

21. The sensor controlling device according to Claim20 further comprising a display unit,
wherein the controlling unit has the display unit display the period variations.

22. A sensor controlling device that receives a vital sign transmitted from a vital sign detection sensor and controls a vital sign detection operation of the vital sign detection sensor, the sensor controlling device comprising:
a control code transmitting unit operable to transmit by lightwaves a control code for making the vital sign detection sensor perform the vital sign detection operation;
a vital sign receiving unit operable to cordlessly receive the vital sign transmitted from the vital sign detection sensor;
a memory unit operable to memorize the vital sign received by the vital sign receiving unit by an amount of a specified time period; and
a controlling unit operable to control the control code transmitting unit transmitting the control code, from when the vital sign detection operation of the vital sign detection sensor starts to when the memory unit finishes memorizing the vital sign by the amount of the specified time period.

23. The sensor controlling device according to Claim22,
wherein the vital sign is transmitted from the vital sign detection sensor by radio, and
the vital sign receiving unit is equipped with an antenna that receives the vital sign transmitted from the vital sign detection sensor by radio.

24. The sensor controlling device according to Claim22,
wherein the vital sign is transmitted from the vital sign detection sensor by lightwaves, and
the vital sign receiving unit includes a light-electricity converting unit operable to receive the vital sign transmitted from the vital sign detection sensor by lightwaves.

25. The sensor controlling device according to Claim22,
wherein the controlling unit has the control code transmitting unit start transmitting the control code at a predetermined time.

26. The sensor controlling device according to Claim22,
wherein the controlling unit has the control code transmitting unit start transmitting the control code when an event occurs.

27. The sensor controlling device according to Claim22 further comprising a touch sensor that a user wearing the vital sign detection sensor touches,
wherein the controlling unit has the control code transmitting unit start transmitting the control code when the user wearing the vital sign detection sensor touches the touch sensor.

28. The sensor controlling device according to Claim27 further comprising:
a battery for supplying power to device components; and
a power supply switching unit operable to connect a power supply channel between the battery and the device components, when the user wearing the vital sign detection sensor touches the touch sensor, synchronously with the touch.

29. The sensor controlling device according to Claim27,
wherein the vital sign is transmitted via the user's body as a transmission medium, and
the vital sign receiving unit includes an interfacing unit operable to receive via the touch sensor the vital sign transmitted from the vital sign detection sensor using the user's body as the transmission medium.

30. The sensor controlling device according to Claim22,
wherein the controlling unit performs statistical processing for the vital sign stored in the memory unit by the amount of the specified time period, and determines the number of pulses per unit of time.

31. The sensor controlling device according to Claim30 further comprising a display unit,
wherein the controlling unit has the display unit display the number of the pulses per unit of time determined by the statistical processing.

32. The sensor controlling device according to Claim30,
wherein the controlling unit further determines period variations of the number of the pulses.

33. The sensor controlling device according to Claim32 further comprising a display unit,
wherein the controlling unit has the display unit display the period variations.

34. A vital sign detection system that comprises a vital sign detection sensor and a sensor controlling device,
wherein the vital sign detection sensor includes a tape that has an adhesive side on which one or more chips are attached and that is elastic,
wherein said one or more chips are firmly fixed on a detection position of a user's body by winding the tape around the detection position, with a vital sign detecting side of said one or more chips contacting the detection position, and
the sensor controlling device receives a vital sign transmitted from the vital sign detection sensor, controls a vital sign detection operation of the vital sign detection sensor, and includes:
a power carrier radio wave transmitting unit operable to transmit a power carrier radio wave when the vital sign detection sensor detects the vital sign;
a vital sign receiving unit operable to cordlessly receive the vital sign transmitted from the vital sign detection sensor;
a memory unit operable to memorize the vital sign received by the vital sign receiving unit by an amount of a specified time period; and
a controlling unit operable to control the power carrier radio wave transmitting unit transmitting the power carrier radio wave, from when the vital sign detection operation of the vital sign detection sensor starts to when the memory unit finishes memorizing the vital sign by the amount of the specified time period,
wherein the tape of the vital sign detection sensor is wound around a human finger, and
the sensor controlling device includes an arm band that is wound around a human arm.

35. A vital sign detection system that comprises a vital sign detection sensor and a sensor controlling device,
wherein the vital sign detection sensor includes a tape that has an adhesive side on which one or more chips are attached and that is elastic,
wherein said one or more chips are firmly fixed on a detection position of a user's body by winding the tape around the detection position, with a vital sign detecting side of said one or more chips contacting the detection position, and
the sensor controlling device receives a vital sign transmitted from the vital sign detection sensor, controls a vital sign detection operation of the vital sign detection sensor, and includes:
a control code transmitting unit operable to transmit by lightwaves a control code for making the vital sign detection sensor perform the vital sign detection operation;
a vital sign receiving unit operable to cordlessly receive the vital sign transmitted from the vital sign detection sensor;
a memory unit operable to memorize the vital sign received by the vital sign receiving unit by an amount of a specified time period; and
a controlling unit operable to control the control code transmitting unit transmitting the control code, from when the vital sign detection operation of the vital sign detection sensor starts to when the memory unit finishes memorizing the vital sign by the amount of the specified time period,
wherein the tape of the vital sign detection sensor is wound around a human finger, and
the sensor controlling device includes an arm band that is wound around a human arm.

36. A vital sign detecting method employed by a vital sign detection sensor that is portable and used attached to a human body,
wherein energy is externally taken in so as to generate direct current power from the energy,
a vital sign of a user is detected while being supplied with the direct current power, and
the vital sign that is detected while being supplied with the direct current power is transmitted cordlessly.

37. A program for a sensor controlling device that receives a vital sign transmitted from a vital sign detection sensor and controls a vital sign detection operation of the vital sign detection sensor, the program having a computer function as the following units of the sensor controlling device:
a power carrier radio wave transmitting unit operable to transmit a power carrier radio wave when the vital sign detection sensor detects the vital sign;
a vital sign receiving unit operable to cordlessly receive the vital sign transmitted from the vital sign detection sensor;
a memory unit operable to memorize the vital sign received by the vital sign receiving unit by an amount of a specified time period; and
a controlling unit operable to control the power carrier radio wave transmitting unit transmitting the power carrier radio wave, from when the vital sign detection operation of the vital sign detection sensor starts to when the memory unit finishes memorizing the vital sign by the amount of the specified time period.

38. A program for a sensor controlling device that receives a vital sign transmitted from a vital sign detection sensor and controls a vital sign detection operation of the vital sign detection sensor, the program having a computer function as the following units of the sensor controlling device:
a control code transmitting unit operable to transmit by lightwaves a control code for making the vital sign detection sensor perform the vital sign detection operation;
a vital sign receiving unit operable to cordlessly receive the vital sign transmitted from the vital sign detection sensor;
a memory unit operable to memorize the vital sign received by the vital sign receiving unit by an amount of a specified time period; and
a controlling unit operable to control the control code transmitting unit transmitting the control code, from when the vital sign detection operation of the vital sign detection sensor starts to when the memory unit finishes memorizing the vital sign by the amount of the specified time period.
